(19) Europäisches Patentamt
European Patent Office
Office européen des brevets

(11) **EP 2 407 150 A1**

(12) **EUROPÄISCHE PATENTANMELDUNG**

(43) Veröffentlichungstag:
**18.01.2012 Patentblatt 2012/03**

(21) Anmeldenummer: **10169915.5**

(22) Anmeldetag: **16.07.2010**

(51) Int Cl.:
*A61K 9/00* (2006.01)     *A61K 9/51* (2006.01)
*A61K 31/78* (2006.01)     *A61K 31/785* (2006.01)
*C08L 33/14* (2006.01)

(84) Benannte Vertragsstaaten:
**AL AT BE BG CH CY CZ DE DK EE ES FI FR GB
GR HR HU IE IS IT LI LT LU LV MC MK MT NL NO
PL PT RO SE SI SK SM TR**
Benannte Erstreckungsstaaten:
**BA ME RS**

(71) Anmelder: **Justus-Liebig-Universität Gießen
35390 Giessen (DE)**

(72) Erfinder:
• **Beck-Broichsitter, Moritz
35039, Marburg (DE)**
• **Schmehl, Thomas
35390, Gießen (DE)**
• **Gessler, Tobias
35435, Wettenberg (DE)**

(74) Vertreter: **Stumpf, Peter
Kerkrader Straße 3
35394 Gießen (DE)**

(54) **Nano- und Mikropolymerpartikel zur Aufrechterhaltung einer niedrigen Oberflächenspannung in der Lunge und zum Schutz des pulmonalen Surfactants**

(57)     Die vorliegende Erfindung stellt biokompatible Nano-, Meso- und Mikropolymerpartikel bereit, welche pathogene Proteine binden können, die in den Lining Layer der Lunge eindringen. Bekannte pathogene Proteine im pulmonalen Lining Layer sind negativ geladen. Sie schädigen das pulmonale Surfactant-System, welches für die Aufrechterhaltung einer niedrigen Oberflächenspannung in der Lunge und damit für eine funktionsfähige Atmung essentiell ist. Erfindungsgemäße Polymerpartikel weisen einen Durchmesser zwischen 20 nm und 10 μm auf, sind wasserunlöslich, haben eine positive Oberflächenladung und eine geringe Oberflächenhydrophobizität. Der isoelektrische Punkt der Partikel ist größer als 5, damit die Partikel im Lining Layer der Lunge als positiv geladene Partikel vorliegen, und zugleich größer als der isoelektrische Punkt des zu bindenden pathogenen Proteins.

Die erfindungsgemäßen Polymerpartikel können beispielsweise über Präzipitatitions- oder Emulsionsverfahren hergestellt werden.

Die erfindungsgemäßen Polymerpartikel können zur Aufrechterhaltung einer niedrigen Oberflächenspannung in der Lunge und zum Schutz des pulmonalen Surfactants verwendet werden.

**EP 2 407 150 A1**

## Beschreibung

Die vorliegende Erfindung stellt Nano-, Meso- und Mikropolymerpartikel bereit, die eine positive Oberflächenladung und ein positives ζ-Potential besitzen. Diese Partikel sind in der Lage, pathogene Proteine zu binden, welche bei bestimmten Erkrankungen in den Lining Layer der Lunge eindringen und dadurch die biophysikalischen Eigenschaften des pulmonalen Surfactants beeinflussen. Auf diese Weise schützen die erfindungsgemäßen Polymerpartikel das pulmonale Surfactant-System.

## Beschreibung des allgemeinen Gebietes der Erfindung

Die vorliegende Erfindung betrifft die Gebiete Innere Medizin, Pharmakologie, Nanotechnologie und Medizintechnik.

## Stand der Technik

Der Alveolarraum von Säugerlungen ist von einem komplexen Surfactantsystem bedeckt, das die Oberflächenspannung reduziert, um ein Kollabieren der Alveolen während des Atmens zu verhindern. Pulmonaler Surfactant wird von Typ II-Pneumozyten sezerniert und enthält ungefähr 90 % Lipide und 10 % Proteine. Die Lipide, die die Alveolaroberflächen bedecken, bestehen vor allem aus Phospholipiden (~80-90 %) und einem kleineren Anteil neutraler Lipide (~so-20 %). Unter den Phospholipiden überwiegen Phosphatidylcholine (~70-80 %) und Phosphatidylgycerole, wobei kleinere Mengen an Phosphatidylinositolen, Phosphatidylserinen und Phosphatidylethanolaminen vorkommen. Etwa die Hälfte der Proteinmasse des alveolären Surfactants besteht aus den Surfactant-assoziierten Proteinen SP-A und SP-D, bei denen es sich um hochmolekulare hydrophile Proteine handelt, sowie SP-B und SP-C, die niedermolekulare hydrophobe Proteine sind. Zahlreiche *in vitro*-Studien beschäftigten sich mit der komplexen Wechselwirkung zwischen Phospholipiden (Phosphatidylcholinen und Phosphatidylglycerolen) und Surfactantproteinen (SP-B und SP-C), die die Abnahme der Oberflächenspannung in der Alveolarregion auf Werte nahe 0 mN/m während der Kompressions-Expansions-Zyklen ermöglichen. Derart extrem niedrige Oberflächenspannungen können nur durch Oberflächenfilme erzielt werden, die reich an Phospholipiden sind. Darüber hinaus weisen diese Monoschichten eine hinreichende Stabilität und Fluidität auf, um den Ersatz individueller Surfactantbestandteile während einer Änderung der Oberfläche der Luft-Wasser-Grenzfläche zu ermöglichen. Während der Kompression des Oberflächenfilms (Exspiration) fördern SP-B und SP-C die Reinigung der Monoschicht, und vor allem Nichtphospholipidverbindungen werden in die Bulkphase zurück befördert ("squeeze out"), wo ein "Oberflächenassoziiertes Reservoir" gebildet wird. Bei Expansion der Alveolaroberfläche (Inspiration) erleichtern SP-B und SP-C den schnellen Wiedereintritt und die Wiederausbreitung der Surfactantlipide, die sich in diesem "Oberflächen-assoziierten Surfactant-Reservoir" ("surface-associated surfactant reservoir") befinden, was für die Limitierung des Anstiegs der Oberflächenspannung essentiell ist.

Das Eindringen von Plasmaproteinen (Albumin) in das Lining Layer der Lunge führt zu einer Beeinflussung der pulmonalen Surfactant-Funktion, wie dies beispielsweise für das Adult Respiratory Distress Syndrome (ARDS) beschrieben wurde (W. Seeger, C. Grube, A. Günther, R. Schmidt: "Surfactant inhibition by plasma proteins: differential sensitivity of various surfactant preparations", Eur Respir J 1993, 6:971-977). Eine gestörte Surfactant-Funktion wird auch bei weiteren pulmonalen und kardialen Erkrankungen beobachtet (A. Günther, C. Siebert, R. Schmidt, S. Ziegler, F. Grimminger, M. Yabut, B. Temmesfeld, D. Walmrath, H. Morr, W. Seeger: "Surfactant alterations in severe pneumonia, acute respiratory distress syndrome, and cardiogenic lung edema", Am J Respir Crit Care Med 1996, 153:176-184; A. Günther, R. Schmidt, F. Nix, M. Yabut-Perez, C. Guth, S. Rosseau, C. Siebert, F. Grimminger, H. Morr, H.G. Velcovsky, W. Seeger: "Surfactant abnormalities in idiopathic pulmonary fibrosis, hypersensitivity pneumonitis and sarcoidosis", Eur Respir J 1999, 14:565-573). Als mögliche Therapiestrategie bei derartigen Erkrankungen wird seit Jahren die Surfactant-Replacement-Therapie evaluiert, bei der synthetischer oder natürlicher Surfactant in die Lunge eingebracht wird. Allerdings löst dieses therapeutische Vorgehen nicht das eigentliche Problem, nämlich das Vorhandensein schädlicher Proteine im Lining Layer der Lunge. Entsprechend kann es zur Beeinträchtigung bzw. zur Inaktivierung auch des therapeutisch in die Lunge eingebrachten Surfactants kommen.

In den letzten Jahren wurden biokompatible Polymerpartikel als mögliche Arzneistoffträger, auch für die pulmonale Applikation, untersucht. Mit Hilfe der direkten Applikation verkapselter Wirkstoffe in die Lunge kann eine lang anhaltende und kontrollierte Wirkstofffreigabe am gewünschten Wirkort erreicht werden, was zu einer verlängerten pharmakologischen Wirkung führt. Des Weiteren gestatten Polymerpartikelformulierungen den Schutz des verkapselten Wirkstoffs vor Abbau sowie das zielgerichtete Ansprechen spezifischer Wirkorte oder Zellpopulationen innerhalb des respiratorischen Systems. Inzwischen wurde in zahlreichen *in vitro*-und *in vivo*-Studien für verschiedene biodegradierbare Polymerpartikel eine gute pulmonale Verträglichkeit gezeigt.

Überraschenderweise können in die Lunge eingebrachte Nano-, Meso- und Mikropolymerpartikel durch Adsorption von in den Lining Layer der Lunge eingedrungenen Plasmaproteinen auf ihre Oberfläche die pulmonale Surfactant-Funktion positiv beeinflussen. Adsorbierte Plasmaproteine sind nicht mehr in der Lage, die Struktur des Surfactants an der Luft-Wasser-Grenzfläche zu stören.

Die Ergebnisse der vorliegenden Erfindung zeigen, dass erfindungsgemäße Nano-, Meso- und Mikropolymerpartikel zur Wiederherstellung und Aufrechterhaltung einer niedrigen Oberflächenspannung in der Lunge und zum Schutz des pulmonalen Surfactants geeignet sind.

**Aufgabe**

Aufgabe der Erfindung ist es, Mittel zur Wiederherstellung und Aufrechterhaltung einer niedrigen Oberflächenspannung in der Lunge und zum Schutz des pulmonalen Surfactants bereitzustellen.

**Lösung der Aufgabe**

Die Aufgabe der Bereitstellung von Mitteln zur Wiederherstellung und Aufrechterhaltung einer niedrigen Oberflächenspannung in der Lunge und zum Schutz des pulmonalen Surfactants wird erfindungsgemäß gelöst durch biokompatible Nano-, Meso- und Mikropolymerpartikel zur Bindung pathogener Proteine, die in den Lining Layer der Lunge eindringen, dadurch gekennzeichnet, dass die Partikel

- einen Durchmesser zwischen 20 nm und 10 μm aufweisen,
- wasserunlöslich sind,
- eine positive Oberflächenladung aufweisen,
- eine geringe Oberflächenhydrophobizität aufweisen, gleichbedeutend mit einem Kontaktwinkel kleiner 120°, gemessen nach der Sessile-drop-Methode,
- einen isoelektrischen Punkt aufweisen, der größer als 5 ist, damit die Partikel unter physiologischen Bedingungen in der Lunge als positiv geladene Partikel vorliegen und der zugleich größer ist als der isoelektrische Punkt des zu bindenden pathogenen Proteins, und
- ein positives ζ-Potential größer als +20 mV aufweisen.

Überraschend wurde gefunden, dass biokompatible Nano-, Meso- und Mikropolymerpartikel mit diesen Eigenschaften in der Lage sind, eine niedrige Oberflächenspannung in der Lunge aufrecht zu erhalten und den pulmonalen Surfactant zu schützen, indem sie pathogene Proteine binden, die in den Lining Layer der Lunge eindringen. Der Lining Layer der Lunge wird auch als Lungenflüssigkeitsfilm bezeichnet.

Nachfolgend werden die erfindungsgemäßen und biokompatiblen Nano-, Meso-und Mikropolymerpartikel kurz als "Polymerpartikel" bezeichnet.

Die natürlichen Surfactantproteine SP-B und SP-C sind vergleichsweise kleine Proteine mit einem Molekulargewicht ≤ 10 kDa. Sie sind sehr hydrophob und besitzen einen isoelektrischen Punkt (IEP) von etwa 10-12, liegen also unter physiologischen Bedingungen positiv geladen vor.

Dagegen sind bekannte pathogene Proteine, z.B. pathogene Plasmaproteine, signifikant größer, besitzen also ein deutlich höheres Molekulargewicht (MW). Im Gegensatz zu den Surfactantproteinen SP-B und SP-C weisen sie einen IEP kleiner als 8 auf und sind daher unter physiologischen Bedingungen negativ geladen. Des Weiteren sind diese pathogenen Proteine deutlich weniger hydrophob als die natürlichen Surfactantproteine. Beispiele für physiologisch relevante pathogene Proteine sind Albumin (IEP: ca. 4,6; MW ca. 66 kDa), Fibrinogen (IEP: ca. 5,8; MW: ca. 340 kDa) und Hämoglobin (IEP: ca. 7,1; MW: ca. 64 kDa). Die isoelektrischen Punkte und Molekulargewichte pathogener Proteine, die in der Lunge auftreten, sind dem Fachmann bekannt.

Die pathogenen Proteine dringen in den Lining Layer der Lunge ein, stören die physiologische Wechselwirkung der natürlichen Surfactantproteine mit Phospholipiden und führen auf diese Weise zu einer erhöhten Oberflächenspannung in der Lunge.

Die erfindungsgemäßen Polymerpartikel weisen einen isoelektrischen Punkt (IEP) auf, der größer als 5 ist, damit sie unter physiologischen Bedingungen in der Lunge als positiv geladene Partikel vorliegen. In einer bevorzugten Ausführung weisen die erfindungsgemäßen Polymerpartikel einen isoelektrischen Punkt auf, der größer als 7 ist. In einer besonders bevorzugten Ausführung weisen die erfindungsgemäßen Partikel einen isoelektrischen Punkt auf, der größer als 9 ist. Des Weiteren ist der isoelektrische Punkt der erfindungsgemäßen Polymerpartikel so zu wählen, dass er größer ist als der IEP des zu bindenden pathogenen Proteins. Dem Fachmann ist bekannt, wie er den isoelektrischen Punkt von Polymeren bestimmen kann, beispielsweise mittels Polyelektrolyt-Titration. Er kann diese Kenntnisse verwenden, ohne den Schutzbereich der Patentansprüche zu verlassen.

Optional können die erfindungsgemäßen Polymerpartikel permanent positiv geladen sein. Dies ist beispielsweise der Fall, wenn sie Polymere mit quartären Stickstoffatomen enthalten.

Die erfindungsgemäßen Polymerpartikel haben einen Durchmesser zwischen 20 nm und 10 μm. Partikel mit

einem Durchmesser von mindestens 200 nm werden von den Makrophagen der Lunge aktiv erkannt, aufgenommen und abgebaut. Die pulmonalen Makrophagen stellen einen effizienten Clearancemechanismus der tiefen Lunge (respirable Bronchien, Alveolarraum) dar. Nanopartikel, die kleiner als 200 nm sind, werden durch unspezifische Mechanismen (passiver Transport, Diffusion, Endozytose, Transzytose etc.) aus dem Lining Layer der tiefen Lunge in Epithelzellen, Makrophagen, Zellen der Immunabwehr, dendritische Zellen, Endothelzellen, oder in das Interstitium entfernt. Die erfindungsgemäßen Polymerpartikel dürfen jedoch nicht größer als 10 μm sein, da größere Partikel nicht mehr effektiv von den Makrophagen der Lunge aufgenommen und abgebaut werden können. In einer bevorzugten Ausführung weisen die erfindungsgemäßen Polymerpartikel einen Durchmesser zwischen 200 nm und 10 μm auf. In einer besonders bevorzugten Ausführung weisen die erfindungsgemäßen Polymerpartikel einen Durchmesser zwischen 200 nm und 6 μm auf.

Die erfindungsgemäßen Polymerpartikel sind wasserunlöslich. Unter wasserunlöslichen Polymeren werden dabei im Sinne der vorliegenden Erfindung solche Polymere verstanden, deren Löslichkeit in Wasser weniger als 0,1 Gewichtsprozent beträgt.

Die erfindungsgemäßen Polymerpartikel weisen eine positive Oberflächenladung und ein positives ζ-Potential größer als +20 mV auf, damit sie aufgrund elektrostatischer Wechselwirkung pathogene Proteine adsorbieren können, während natürliche Surfactantproteine nicht adsorbiert werden. In einer bevorzugten Ausführung weisen die erfindungsgemäßen Polymerpartikel eine positive Oberflächenladung und ein positives ζ-Potential größer als +40 mV auf. In einer besonders bevorzugten Ausführung weisen die erfindungsgemäßen Polymerpartikel eine positive Oberflächenladung und ein positives ζ-Potential größer als +60 mV auf. Bei Partikeln mit einer positiven Oberflächenladung handelt es sich um kationische Partikel.

Die Adsorption der eher hydrophilen pathogenen Proteine wird zudem durch die geringe Oberflächenhydrophobizität der erfindungsgemäßen Polymerpartikel begünstigt. Dagegen werden die hydrophoben Surfactantproteine nicht adsorbiert. Erfindungsgemäß wird unter "geringer Oberflächenhydrophobizität" verstanden, dass der Kontaktwinkel, gemessen nach der Sessile-drop-Methode, kleiner als 120° Grad ist. In einer bevorzugten Ausführung weisen die erfindungsgemäßen Polymerpartikel eine Oberflächenhydrophobizität auf, dass der Kontaktwinkel, gemessen nach der Sessile-drop-Methode, kleiner als 90° Grad ist. In einer besonders bevorzugten Ausführung weisen die erfindungsgemäßen Polymerpartikel eine Oberflächenhydrophobizität auf, dass der Kontaktwinkel, gemessen nach der Sessile-drop-Methode, kleiner als 60° Grad ist.

Man erkennt, dass die durch die erfindungsgemäßen Polymerpartikel bewirkte Adsorption pathogener Proteine auf rein physikalischen Wechselwirkungsprozessen zwischen Partikel und Protein beruht. Es ist für den Fachmann offensichtlich, dass alle Partikel, die die Merkmale des Anspruchs 1 aufweisen, zur Adsorption pathogener Proteine im Lining Layer der Lunge geeignet sind.

Geeignete Monomere, aus denen die erfindungsgemäßen Polymerpartikel hergestellt werden können, sind beispielsweise, aber nicht erschöpfend, Acrylate, Methacrylate, Butylmethacrylate, (2-Dimethylaminoethyl)-methacrylat, Amine, Amide, Acetale, Polyester, Ketale, Anhydride und Saccharide. Dabei kann es sich um ein Homopolymer, Copolymer, Blockpolymer, Pfropfcopolymer, Sternpolymer, Kammpolymer, hochverzweigtes Polymer, statistisches Polymer oder Dendrimer handeln.

Erfindungsgemäße Polymerpartikel weisen eine positive Oberflächenladung auf. Dies bedeutet, dass das Polymer entweder selbst positive Ladungen trägt (z.B. kationische Acrylate, kationische Kammpolymere) und über eine bekannte Herstellungsmethode (z.B. Nanopräzipitation, Emulsionsmethode) zu Partikeln verarbeitet wird. Des Weiteren können Polymere verwendet werden, die ungeladen vorliegen oder negative Ladungen tragen und über eine bekannte Herstellungsmethode (z.B. Nanopräzipitation, Emulsionsmethode) zu Partikeln verarbeitet werden können. Die positive Ladung der Partikel wird durch Anwesenheit kationischer Emulgatoren (z.B. Cetylpyridiniumchlorid) während der Herstellung erzeugt. Außerdem lassen sich Partikel nach der Herstellung durch zusätzliche Schritte (Coating mit positiv geladenen Coatingsubstanzen wie Chitosan, DEAE-Dextran, DEA-PA, PEI) mit einer positiven Oberflächenladung versehen. Dabei steht DEAE für eine Diethylaminoethylgruppe, DEA-PA für Diethylamino-Polyamid und PEI für Polyethylenimin.

In einer bevorzugten Ausführungsform bestehen die erfindungsgemäßen Polymerpartikel aus Poly-(butylmethacrylat)-co-(2-dimethylaminoethyl)-methacrylat-co-methylmethacrylat. Dieses Terpolymer ist dem Fachmann bekannt und kann im Rahmen der vorliegenden Erfindung zur Herstellung der erfindungsgemäßen Polymerpartikel verwendet werden. Das Verhältnis der drei Monomersorten Butylmethacrylat, 2-(Dimethylaminoethyl)-methacrylat und Methylmethacrylat kann dabei erfindungsgemäß variieren, solange die resultierenden Partikel die Merkmale des Anspruchs 1 aufweisen. Dem Fachmann ist bekannt, wie er feststellen kann, welche Terpolymerzusammensetzungen diese Bedingung erfüllen. Er kann also ohne großen Aufwand und ohne den Schutzbereich der Patentansprüche zu verlassen geeignete Terpolymere ermitteln.

In einer besonders bevorzugten Ausführungsform liegen Butylmethacrylat, 2-(Dimethylaminoethyl)-methacrylat und Methylmethacrylat im Verhältnis 1:2:1 (w/w/w) vor. Ein solches Terpolymer ist unter dem Namen Eudragit® E100 bekannt.

Die erfindungsgemäßen Polymerpartikel können beispielsweise mittels Nanopräzipitation oder mit Hilfe der Emulsionsmethode hergestellt werden. Des Weiteren können Nanopartikel auch mittels Aussalzen oder Polymerisation und Mikropartikel mittels Sprühtrocknung hergestellt werden.

Werden die Partikel mittels Nanopräzipitation hergestellt, so wird eine 0,1 bis 10 %-ige Lösung (w/v) des Polymers in einem ersten, polar aprotischen Lösungsmittel hergestellt und anschließend in einem zweiten Lösungsmittel gefällt. Das erste Lösungsmittel muss dabei das Polymer lösen und mit dem zweiten Lösungsmittel vollständig oder teilweise mischbar sein, wobei das zweite Lösungsmittel das Polymer nicht löst. Geeignete polar aprotische Lösungsmittel sind beispielsweise Aceton, Acetonitril, Tetrahydrofuran, Dimethylsulfoxid, Trichlormethan und Ethylenamin. Vorteilhaft wird als zweites Lösungsmittel Wasser eingesetzt. Die Fällung kann erfolgen, indem die Polymerlösung in das zweite Lösungsmittel gegeben oder gegen dieses Lösungsmittel dialysiert wird. Anschließend wird das organische Lösungsmittel entfernt und die Partikel in Suspension erhalten.

Die Auswahl des ersten Lösungsmittels richtet sich dabei nach der Herstellungsmethode. Es sind prinzipiell solche Lösungsmittel als erste Lösungsmittel geeignet, die das Polymer lösen und mit dem zweiten Lösungsmittel vollständig oder teilweise mischbar sind. Definierte Durchmesser der erfindungsgemäßen Polymerpartikel sowie enge Größenverteilungen lassen sich über die gewählte Herstellungsmethode, die Polymerkonzentration in der organischen Phase, die Rührgeschwindigkeit, die Mischungsgeschwindigkeit und die Volumenverhältnisse einstellen. Des Weiteren kann der Polymerlösung optional ein Tensid zugesetzt werden, beispielsweise ein anionisches oder nichtionisches Tensid. Auch mit Hilfe einer Tensidzugabe lassen sich der Durchmesser der erfindungsgemäßen Polymerpartikel sowie die Größenverteilung definiert einstellen. Die genannten Methoden zur Einstellung des Durchmessers und der Größenverteilung sind dem Fachmann bekannt und können, ohne den Schutzbereich der Patentansprüche zu verlassen, angewendet werden.

Die Erfindung ist nicht auf eine der vorbeschriebenen Ausführungsformen beschränkt, sondern in vielfältiger Weise abwandelbar.

Sämtliche aus den Ansprüchen, der Beschreibung und den Zeichnungen hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

Die erfindungsgemäßen Polymerpartikel sind vorteilhaft mit piezoeleketrischen, Düsen-, Ultraschall-Aerosolgeneratoren, Soft-Mist-Inhalern, Metered Dose Inhalern oder Dry Powder Inhalern verneblbar, d.h. die Applikation in die Lunge erfolgt durch Inhalation eines Aerosols (Suspension, Pulver) mit Hilfe eines Verneblers. Für diese Applikation ist es vorteilhaft, wenn die erfindungsgemäßen Polymerpartikel einen Durchmesser kleiner 6 μm aufweisen, um in die tiefe Lunge gelangen zu können.

Eine weitere Möglichkeit der Applikation in die Lunge besteht in der Instillation, beispielsweise über einen Katheter, ein Bronchoskop oder einen Beatmungszugang (z.B. Tubus oder Trachealkanüle).

Die erfindungsgemäßen Polymerpartikel können zur Herstellung eines pharmazeutischen Mittels zur Prävention und/oder Behandlung von Lungenerkrankungen verwendet werden, die mit einem Anstieg der Oberflächenspannung in der Lunge und einer Schädigung des pulmonalen Surfactants einhergehen. Die erfindungsgemäßen Polymerpartikel dienen dabei der Wiederherstellung und Aufrechterhaltung einer niedrigen Oberflächenspannung in der Lunge und dem Schutz des pulmonalen Surfactants.

So können die erfindungsgemäßen Polymerpartikel zur Herstellung von Mitteln zur Behandlung oder Diagnose folgender Erkrankungen eingesetzt werden: Neonatal Respiratory Distress Syndrome, Acute/Adult Respiratory Distress Syndrome (ARDS), Acute Lung Injury (ALI), Lungeninfektionen, Pneumonie, pulmonale Hypertonie, kardiogenes Lungenödem, Asthma, Chronic Obstructive Pulmonary Disease (COPD) / Emphysem, interstitielle Lungenerkrankungen, Lungentumoren, toxische Alveolitis, alveoläres Hämorrhagie-Syndrom, Mukoviszidose, idiopathische pulmonale Hämosiderose, Kollagenkrankheiten, Vaskulitiden, Pneumokoniosen, eosinophile Lungeninfiltrate, Strahlenschäden, hereditäre oder kongenitale Lungenerkrankungen.

Die Wirkung der erfindungsgemäßen Polymerpartikel ist dabei rein physikalischer Natur, indem wie oben beschrieben pathogene Proteine adsorbiert werden. Anschließend werden die mit pathogenen Proteinen beladenen Polymerpartikel von Makrophagen der Lunge eliminiert oder durch unspezifische Clearance-Mechanismen aus dem pulmonalen Lining Layer entfernt.

**Ausführungsbeispiele**

**Materialien**

Das kationische Polymer, nämlich Poly(butylmethacrylat)-co-(2-dimethylaminoethyl)-methacrylat-co-methylmethacrylat) 1:2:1 (Eudragit® E100) wurde von Roehm (Darmstadt, Deutschland) bezogen. Cytochrom C (aus Rinderherz, 95 %) und bovines Serumalbumin (BSA) wurden von Sigma-Aldrich (Steinheim, Deutschland) bezogen. Alle anderen in diesen Experimenten verwendeten Chemikalien und Lösungsmittel wiesen den höchsten kommerziell erhältlichen Reinheitsgrad auf.

**Methoden**

*1. Herstellung der Nanopartikel*

Die Nanopartikel wurden in Anlehnung an die in Hyun-Jeong Jeon, Young-II Jeong, Mi-Kyeong Jang, Young-Hoon Park, Jae-Woon Nah: "Effect of solvent on the preparation of surfactant-free poly(DL-lactide-co-glycolide) nanoparticles and norfloxacin release characteristics", Int J Pharm 2000, 207:99-108, beschriebene Methode hergestellt. Im Rahmen der vorliegenden Erfindung wurden 200 mg Polymer (Eudragit® E100) in 10 ml Aceton gelöst. Dann wurden 10 ml Polymerlösung in einen Dialyseschlauch (Porengröße 10 kDa) gegeben und für 24 h gegen Aqua dest. dialysiert. Die Partikel wurden unmittelbar nach ihrer Herstellung charakterisiert und verwendet.

Charakterisierung der Nanopartikel

Die gemäß Methoden, Punkt 1 hergestellten Nanopartikel wurden mit Hilfe der nachfolgend unter Punkt 2 bis 4 beschriebenen Methoden charakterisiert.

*2. Rasterelektronenmikroskopie (SEM)*

Ein Tropfen der verdünnten Nanopartikel-Suspension wurde auf einen Silizium-Wafer aufgebracht. Nachfolgend wurden alle Proben im Vakuum getrocknet und mittels eines Gatan Alto 2500 Sputter Coaters (Gatan GmbH, Muenchen, Deutschland) mit Platin beschichtet. Die Morphologie der Nanopartikel wurde bei 2 - 5 kV mittels eines Rasterelektronenmikroskops (JSM-7500F, JEOL, Eching, Deutschland) untersucht.

*3. Messung von Größe und ζ-Potential*

Hydrodyamischer Durchmesser und Größenverteilung der erhaltenen Nanopartikel wurden mittels dynamischer Lichtstreuung (dynamic light scattering, DLS) gemessen; das ζ-Potential wurde durch Laser-Doppler-Anemometrie (LDA) unter Verwendung eines Zetasizers NanoZS/ZEN3600 (Malvern Instruments, Herrenberg, Deutschland) bestimmt. Alle Versuche wurden bei einer Temperatur von 25 °C und mit Proben in geeigneter Verdünnung durchgeführt. Für die DLS wurden die Proben mit filtriertem und zweifach destilliertem Wasser verdünnt; zur Verdünnung der Proben für die LDA wurde 1,56 mM NaCl verwendet. Alle Messungen wurden in Dreifachbestimmung und unmittelbar nach der Herstellung der Nanopartikel mit mindestens 10 Durchgängen durchgeführt.

*4. Adsorption von Proteinen an Nanopartikeln*

Zur Messung der Adsorption von Cytochrom C und BSA an Nanopartikeln wurden Nanopartikelsuspensionen mit definierten Konzentrationen mit definierten Mengen des Modellproteins Cytochrom C (IEP = 10, MW = 12.3 kDa) und des pathogenen Modellproteins BSA (IEP = ca. 4,6, MW = ca. 66 kDa) für 3 h bei 25 °C inkubiert. Die Menge des auf den Nanopartikeln adsorbierten Proteins wurde als Differenz zwischen der Menge des zu der Nanopartikelsuspension zugegebenen Proteins und der Menge des nicht absorbierten Proteins bestimmt, das in der wässrigen Phase verblieben war. Nach der Inkubation wurden die Proben für 2 h bei 16.000 x g zentrifugiert (Centrifuge 5418, Eppendorf, Hamburg, Deutschland), und das nicht gebundene Protein im Überstand wurde über den Extinktionskoeffizienten von Cytochrom C bzw. BSA bestimmt. Für jede Proteinkonzentration wurde eine Kontrollprobe ohne Nanopartikel hergestellt, um den Proteinverlust während der Inkubationszeit zu ermitteln. Der Proteinbeladungsgrad wurde wie folgt berechnet:

$$Proteinbeladungsgrad = \frac{Gesamtmenge\ Protein - freies\ Protein}{Gesamtmenge\ Protein} \cdot 100\ [\%].$$

Ergebnisse

*1. Eigenschaften der Nanopartikel*

Die physikochemischen Eigenschaften der Nanopartikel, die gemäß "Methoden, Punkt 3" bestimmt wurden, sind in Tabelle 1 aufgelistet.

Tab. 1: Eigenschaften der Nanopartikelformulierungen, die gemäß "Methoden, Punkt 1" hergestellt wurden

| Nanopartikelformulierung | Größe [nm][1] | PDI[1] | ζ-Potential [mV] |
|---|---|---|---|
| Eudragit® E100 | 486 | 0,070 | +53,7 |
| [1]: Die Werte wurden mittels dynamischer Lichtstreuung (DLS) bestimmt. PDI: Polydispersitätsindex | | | |

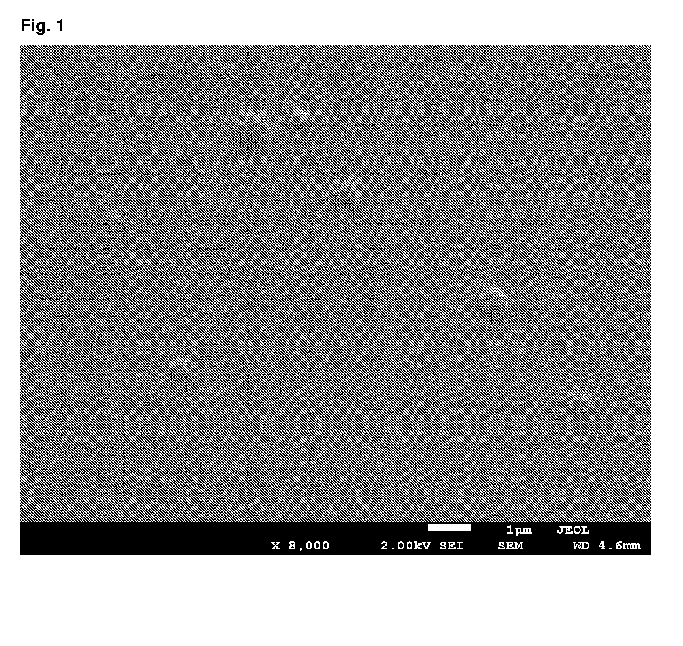

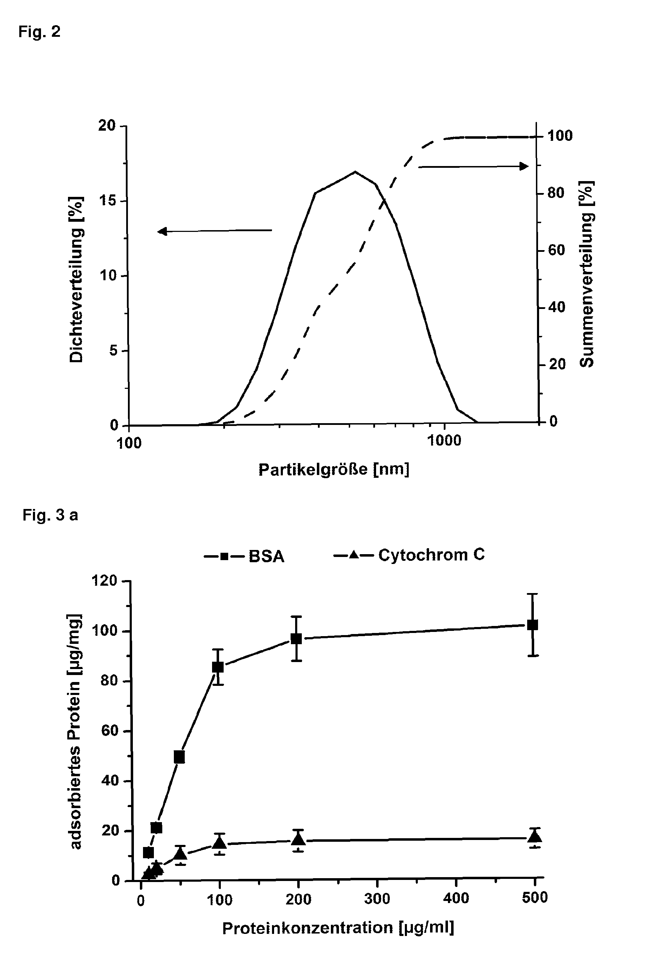

Zur Bestimmung von Struktur, Größe und Größenverteilung der verwendeten Nanopartikelformulierung wurden Rasterelektronenmikroskopie-Messungen (SEM) durchgeführt (Fig. 1). Die Nanopartikel wiesen nahezu Kugelgestalt auf. Größe und Größenverteilung der Nanopartikelformulierungen wurden des Weiteren mittels dynamischer Lichtstreuung (DLS) ermittelt. Typische Kurven der Dichte und Summenpartikelgrößenverteilung gemäß DLS sind in Fig. 2 dargestellt. Sie belegen für alle Formulierungen eine mittlere Größe von etwa 500 nm mit engen Größenverteilungen. Die Daten aus den Rasterelektronenmikroskopie-Messungen (SEM) stimmen gut mit denjenigen aus der dynamischen Lichtstreuung (DLS) überein. Die Oberflächenladung (ζ-Potential) der Eudragit® E100-Nanopartikel wurde über Laser-Doppler-Anemometrie bestimmt, das ζ-Potential betrug +53,7 mV.

*2. Adsorption von Cytochrom C und BSA auf Nanopartikeln*

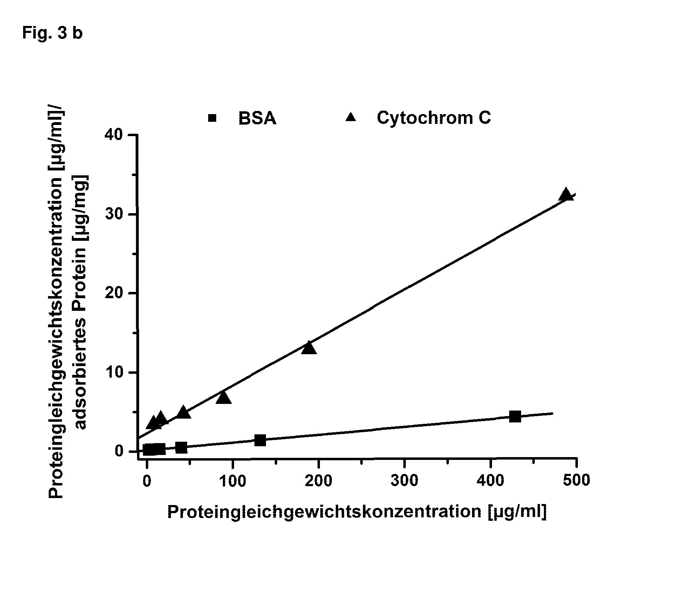

Um die Adsorption von Surfactant-assoziierten Proteinen auf der Oberfläche verschiedener Nanopartikelformulierungen zu simulieren, wurde das positiv geladene Modellprotein Cytochrom C verwendet (Fig. 3). BSA wurde als Modellprotein für ein pathogenes Protein im Lining Layer gewählt. Wie in den Adsorptionsisothermen in Fig. 3 a gezeigt, wo die absorbierte Menge von Cytochrom C beziehungsweise BSA ($\Gamma$) als Funktion der Cytochrom C- bzw. BSA-Konzentration ($c_0$) aufgetragen ist, betrugen die Werte für die Menge des auf der Oberfläche der Nanopartikel adsorbierten Cytochrom C bis zu ~15 µg/mg Polymer (n = 3) bzw. bis zu ~100 µg/mg Polymer (n = 3) im Falle von BSA Die Adsorptionsdaten wurden anschließend über den untersuchten Konzentrationsbereich an das Langmuir-Adsorptionsmodell angepasst, das beschrieben wird durch

$$\frac{c_e}{\Gamma} = \frac{1}{b \cdot \Gamma_m} + \frac{c_e}{\Gamma_m}$$

wobei $\Gamma$ die Menge des adsorbierten Proteins darstellt, $c_e$ die Gleichgewichtsproteinkonzentration im Inkubationsmedium ist, b einen Koeffizienten darstellt, der sich auf die Affinität zwischen Nanopartikeln und Protein bezieht, und $\Gamma_m$ die maximale Adsorptionskapazität darstellt. Die Ausgleichsgeraden sind in Fig. 3b abgebildet, die berechneten Parameter für die Langmuir-Gleichung sind in Tab. 2 dargestellt. Die Daten zeigten die Bildung einer monomolekularen Schicht der Proteine auf der Oberfläche der Nanopartikel ($R^2$ >0.99). Die Affinität (b) und damit die Gibbs'sche freie Adsorptionsenergie ($\Delta G^0$) der Modellproteine zu den Nanopartikeloberflächen hingen von der Art des eingesetzten Proteins ab. Während für das positiv geladene Cytochrom C nur eine geringfügige Affinität beobachtet wurde (b = 0,031 ± 0,012 ml/mg (Mittelwert ± S.D., n = 3) und $\Delta G^0$ = +1,7 ± 1,5 kJ/mol (Mittelwert ± S.D., n = 3)), war die Affinität für BSA deutlich höher (b = 0,070 ± 0,026 ml/mg (Mittelwert ± S.D., n = 3) und $\Delta G^0$ = -4,8 ± 0,7 kJ/mol (Mittelwert ± S.D., n = 3)).

Tabelle 2: Langmuir-Parameter und freie Energie für die Adsorption von BSA und Cytochrom C auf Nanopartikeln, die gemäß "Methoden, Punkt 1" hergestellt wurden

| Protein | Maximale Adsorptions-kapazität $\Gamma_m$ (µg/mg) | Affinitäts-konstante (ml/mg) | $K_a$ | $\Delta G^0$ (kJ/mol) | $R^2$ |
|---|---|---|---|---|---|
| BSA | 105,9 ± 14,7 | 0,070 ± 0,02 | 7,2 ± 1,9 | -4,8 ± 0,7 | 0,9989 ± 0,0010 |

(fortgesetzt)

| Protein | Maximale Adsorptions-kapazität $\Gamma_m$ ($\mu$g/mg) | Affinitäts-konstante (ml/mg) | $K_a$ | $\Delta G^0$ (kJ/mol) | $R^2$ |
|---|---|---|---|---|---|
| Cytochrom C | 17,2 ± 3,7 | 0,031 ± 0,01 | 0,6 ± 0,3 | +1,7 ± 1,5 | 0,9948 ± 0,0014 |

Die Werte sind als Mittelwerte ± Standardabweichung (S.D.) angegeben (n = 3). $\Gamma_m$: maximale Adsorptionskapazität
b: Affinitätskonstante von Cytochrom C zu den Nanopartikeln
$K_a$: Gleichgewichtsassoziationskonstante ($K_a = \Gamma_m b$)
$\Delta G^0$: Gibbs'sche freie Energie ($\Delta G^0 = - RTlnK_a$)

*Schlussfolgerung*

Erkenntnisse über die Wechselwirkungsmechanismen der Polymerpartikel mit Surfactantbestandteilen und Plasmaproteinen können für die gezielte Herstellung und Optimierung kolloidaler Carrier für die Therapie pulmonaler Krankheiten verwendet werden.

Die Adsorption Surfactant-assoziierter Proteine auf der Oberfläche von Polymernanopartikeln führt zu einer Verschlechterung der pulmonalen Surfactant-Funktion. Adsorbierte Surfactantproteine sind nicht mehr in der Lage, die Struktur des Surfactants an der Luft-Wasser-Grenzfläche zu organisieren und können daher auch nicht die Oberflächenspannung in ähnlicher Weise verringern (Ausmaß und zeitlicher Verlauf) wie das native Surfactantmaterial.

Das Eindringen von Plasmaproteinen (z. B. Albumin) in den Lining Layer der Lunge führt zu einer Beeinträchtigung der pulmonalen Surfactant-Funktion, wie dies beispielsweise für das Adult Respiratory Distress Syndrome (ARDS) beschrieben wurde (W. Seeger, C. Grube, A. Günther, R. Schmidt: "Surfactant inhibition by plasma proteins: differential sensitivity of various surfactant preparations", Eur Respir J 1993, 6:971-977). Die Adsorption von in den Lining Layer der Lunge eingedrungenen Plasmaproteine auf der Oberfläche von Polymernanopartikeln ermöglicht es letzteren, die pulmonale Surfactant-Funktion positiv zu beeinflussen. Adsorbierte Plasmaproteine sind nicht mehr in der Lage, die Struktur des Surfactants an der Luft-Wasser-Grenzfläche zu stören.

Die erfindungsgemäßen biokompatiblen Nano-, Meso- und Mikropolymerpartikel sind in der Lage, schädliche Proteine zu eliminieren, ohne die physiologisch relevanten Surfactant-assoziierten Proteine aus dem Lining Layer der Lunge zu entfernen. Auf diese Weise schützen die erfindungsgemäßen Polymerpartikel das pulmonale Surfactant-System.

**Abbildungslegenden**

**Fig.** 1 Rasterelektronenmikroskopisches Bild der Eudragit® E100-Nanopartikel mit einer mittleren Partikelgröße von etwa 500 nm

**Fig. 2** Kurven zur Partikelgrößenverteilung aus der dynamischen Lichtstreuung (DLS) für frisch hergestelltes Eudragit® E100-Nanopartikel mit einer mittleren Partikelgröße von etwa 500 nm. Die durchgezogene Linie stellt die Dichteverteilung der Partikelgröße, die gestrichelte Linie die Summenverteilung der Partikelgröße dar.

**Fig. 3** Adsorptionsfähigkeit (r) der Eudragit® E100-Nanopartikel mit einer mittleren Partikelgröße von etwa 500 nm für BSA und Cytochrom C nach Inkubation der Partikel mit den jeweiligen Proteinen bei verschiedenen Konzentrationen (a) und an das Langmuir-Modell angepasste BSA- und Cytochrom C-Adsorptionsdaten (b). Bei den durchgezogenen Linien in (b) handelt es sich um die Ausgleichsgeraden der Messdaten. Die Werte in (a) sind als Mittelwert ± Standardabweichung dargestellt (n =3).

**Patentansprüche**

1. Biokompatible Nano-, Meso- und Mikropolymerpartikel zur Bindung pathogener Proteine, die in den Lining Layer der Lunge eindringen, **dadurch gekennzeichnet, dass** die Partikel

   - einen Durchmesser zwischen 20 nm und 10 $\mu$m aufweisen,

- wasserunlöslich sind,
- eine positive Oberflächenladung aufweisen,
- eine geringe Oberflächenhydrophobizität aufweisen, gleichbedeutend mit einem Kontaktwinkel kleiner 120°, gemessen nach der Sessile-drop-Methode,
- einen isoelektrischen Punkt aufweisen, der größer als 5 ist, damit die Partikel unter physiologischen Bedingungen in der Lunge als positiv geladene Partikel vorliegen und der zugleich größer ist als der isoelektrische Punkt des zu bindenden pathogenen Proteins, und
- ein positives ζ-Potential größer als +20 mV aufweisen.

2. Polymerpartikel gemäß Anspruch 1, **dadurch gekennzeichnet, dass** die Monomere, aus denen die erfindungsgemäßen Polymerpartikel hergestellt werden, ausgewählt sind aus Acrylaten, Methacrylaten, Butylmethacrylaten, (2-Dimethylaminoethyl)-methacrylaten, Aminen, Amiden, Acetalen, Polyestern, Ketalen, Anhydriden und Sacchariden.

3. Polymerpartikel gemäß Anspruch 2, **dadurch gekennzeichnet, dass** es sich um ein Homopolymer, Copolymer, Blockpolymer, Pfropfcopolymer, Sternpolymer, Kammpolymer, hochverzweigtes Polymer, statistisches Polymer oder Dendrimer handelt.

4. Polymerpartikel gemäß Anspruch 1, **dadurch gekennzeichnet, dass** sie aus Poly-(butylmethacrylat)-co-(2-dimethylaminoethyl)-methacrylat-co-methylmethacrylat bestehen.

5. Polymerpartikel gemäß Anspruch 4, **dadurch gekennzeichnet, dass** im Poly-(butylmethacrylat)-co-(2-dimethylaminoethyl)-methacrylat-co-methylmethacrylat Butylmethacrylat, 2-(Dimethylaminoethyl)-methacrylat und Methylmethacrylat im Verhältnis 1:2:1 (w/w/w) vorliegen.

6. Polymerpartikel gemäß einem der Ansprüche 1 bis 5, **dadurch gekennzeichnet, dass** sie vorzugsweise einen Durchmesser zwischen 200 nm und 10 μm aufweisen, um aktiv von Alveolarmakrophagen phagozytiert werden zu können, damit sie aus dem Lining Layer des unteren Respirationstrakts entfernt werden.

7. Polymerpartikel gemäß einem der Ansprüche 1 bis 6, **dadurch gekennzeichnet dass** sie besonders bevorzugt einen Durchmesser zwischen 200 nm und 6 μm aufweisen, um auch inhalativ in die Lunge appliziert werden zu können.

8. Polymerpartikel gemäß einem der Ansprüche 1 bis 7, **dadurch gekennzeichnet dass** sie mit piezoeleketrischen, Düsen-, Ultraschall-Aerosolgeneratoren, Soft-Mist-Inhalern, Metered Dose Inhalern oder Dry Powder Inhalern vernebelbar sind.

9. Verfahren zur Herstellung von Polymerpartikeln gemäß einem der Ansprüche 1 bis 5, **gekennzeichnet durch** die Schritte

   a) Lösen des Polymers in einem ersten, polar aprotischen Lösungsmittel,
   b) Ausfällen von Partikeln, indem die Polymerlösung in ein zweites Lösungsmittel gegeben oder gegen dieses dialysiert wird, wobei erstes und zweites Lösungsmittel vollständig oder teilweise miteinander mischbar sind, aber das zweite Lösungsmittel das Polymer nicht löst,
   c) Entfernen des organischen Lösungsmittels und Erhalten der Partikel in Suspension.

10. Verwendung von Polymerpartikeln gemäß einem der Ansprüche 1 bis 8 zur Herstellung eines pharmazeutischen Mittels zur Prävention und/oder Behandlung von Lungenerkrankungen, die mit einem Anstieg der Oberflächenspannung in der Lunge und einer Schädigung des pulmonalen Surfactants einhergehen.

11. Verwendung von Polymerpartikeln gemäß einem der Ansprüche 1 bis 8 zur Herstellung eines pharmazeutischen Mittels zur Prävention und/oder Behandlung von Neonatal Respiratory Distress Syndrome, Acute/Adult Respiratory Distress Syndrome (ARDS), Acute Lung Injury (ALI), Lungeninfektionen, Pneumonie, pulmonaler Hypertonie, kardiogenem Lungenödem, Asthma, Chronic Obstructive Pulmonary Disease (COPD) / Emphysem, interstitiellen Lungenerkrankungen, Lungentumoren, toxischen Alveolitiden, alveolärem Hämorrhagie-Syndrom, Mukoviszidose, idiopathischer pulmonaler Hämosiderose, Kollagenkrankheiten, Vaskulitiden, Pneumokoniosen, eosinophilen Lungeninfiltraten, Strahlenschäden, hereditären oder kongenitalen Lungenerkrankungen.

**Fig. 1**

Fig. 2

Fig. 3 a

**Fig. 3 b**

Europäisches
Patentamt

European
Patent Office

Office européen
des brevets

## EUROPÄISCHER RECHERCHENBERICHT

Nummer der Anmeldung

EP 10 16 9915

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 99/33558 A1 (UNIV GENEVE [CH]; QUINTANAR GUERRERO DAVID [MX]; ALLEMANN ERIC [CH]; G) 8. Juli 1999 (1999-07-08) * Beispiele 1-3; Tabelle 1 * * Beispiel comparative; Tabelle 1 * ----- | 1-9 | INV. A61K9/00 A61K9/51 A61K31/78 A61K31/785 C08L33/14 |
| X | WO 01/02087 A1 (UNIV GENEVE LABORATOIRE DE PHA [CH]; QUINTANAR GUERRERO DAVID [MX]; AL) 11. Januar 2001 (2001-01-11) * Beispiele 1-3,12-19; Tabelle 1 * ----- | 1-9 | |
| X | US 2005/123596 A1 (KOHANE DANIEL S [US] ET AL) 9. Juni 2005 (2005-06-09) * Absätze [0096], [0098], [0101], [0120], [0165]; Ansprüche 20,43; Beispiele 1-3 * ----- | 1-8,10, 11 | |
| X | LIU, T.; DONALD, A. M.; ZHANG, Z.: "Novel manipulation in environmental scanning electron microscope for measuring mechanical properties of single nanoparticles" MATERIALS SCIENCE AND TECHNOLOGY, [Online] Bd. 21, Nr. 3, 31. März 2005 (2005-03-31), Seiten 289-294, XP002597971 DOI: 10.1179/174328405X29230 Gefunden im Internet: URL:http://www.bss.phy.cam.ac.uk/pubs/file s/764.pdf> [gefunden am 2010-08-25] * Seite 290, Absatz Materials and methods; Abbildungen 4,6 * ----- -/-- | 1-9 | RECHERCHIERTE SACHGEBIETE (IPC) A61K C08L |

Der vorliegende Recherchenbericht wurde für alle Patentansprüche erstellt

| Recherchenort | Abschlußdatum der Recherche | Prüfer |
|---|---|---|
| München | 7. September 2010 | Schmitt, Johannes |

Europäisches
Patentamt

European
Patent Office

Office européen
des brevets

**EUROPÄISCHER RECHERCHENBERICHT**

Nummer der Anmeldung

EP 10 16 9915

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | R. PIGNATELLO, C. BUCOLO, G. PUGLISI: "Ocular tolerability of Eudragit RS100® and RL100® nanosuspensions as carriers for ophthalmic controlled drug delivery" JOURNAL OF PHARMACEUTICAL SCIENCES, Bd. 91, Nr. 12, 26. September 2002 (2002-09-26), Seiten 2636-2641, XP002597977 online DOI: 10.1002/jps.10227 * Seiten 2637-263, Absatz Material and Methos; Beispiele RL, RS; Tabelle 1 * ----- | 1-3,8,9 | |
| X | WO 2004/046202 A2 (TRANSMIT TECHNOLOGIETRANSFER [DE]; JUSTUS LIEBIG UNI GIESSEN [DE]; UNI) 3. Juni 2004 (2004-06-03) * Tabelle 3, Beispiele 0, 25, 50 und 100 mikrogramm; Seite 9, Zeile 5 - Seite 10, Zeile 7; Anspruch 16; Beispiele 2,3 * ----- | 1-3,6-11 | |
| A | WO 01/76619 A1 (BYK GULDEN LOMBERG CHEM FAB [DE]; HAEFNER DIETRICH [DE]; KELLER ANDREA) 18. Oktober 2001 (2001-10-18) * das ganze Dokument * ----- | 1-11 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 7. September 2010 | Schmitt, Johannes |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
...................................................................
& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**          EP 10 16 9915

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-09-2010

| Im Recherchenbericht angeführtes Patentdokument | Datum der Veröffentlichung | Mitglied(er) der Patentfamilie | Datum der Veröffentlichung |
|---|---|---|---|
| WO 9933558 A1 | 08-07-1999 | AU 5860798 A | 19-07-1999 |
| WO 0102087 A1 | 11-01-2001 | AU 5156799 A | 22-01-2001 |
| US 2005123596 A1 | 09-06-2005 | US 2005244504 A1 | 03-11-2005 |
| WO 2004046202 A2 | 03-06-2004 | AU 2003294626 A1 | 15-06-2004 |
|  |  | CA 2545877 A1 | 03-06-2004 |
|  |  | DE 10253623 A1 | 03-06-2004 |
|  |  | EP 1562559 A2 | 17-08-2005 |
|  |  | US 2006127485 A1 | 15-06-2006 |
| WO 0176619 A1 | 18-10-2001 | AT 323505 T | 15-05-2006 |
|  |  | AU 6587601 A | 23-10-2001 |
|  |  | CA 2405811 A1 | 18-10-2001 |
|  |  | DE 60118898 T2 | 16-11-2006 |
|  |  | DK 1276495 T3 | 14-08-2006 |
|  |  | EP 1276495 A1 | 22-01-2003 |
|  |  | ES 2262656 T3 | 01-12-2006 |
|  |  | JP 2003530356 T | 14-10-2003 |
|  |  | PT 1276495 E | 31-08-2006 |
|  |  | SI 1276495 T1 | 31-10-2006 |
|  |  | US 2003158087 A1 | 21-08-2003 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **W. SEEGER ; C. GRUBE ; A. GÜNTHER ; R. SCHMIDT.** Surfactant inhibition by plasma proteins: differential sensitivity of various surfactant preparations. *Eur Respir J,* 1993, vol. 6, 971-977
- **A. GÜNTHER ; C. SIEBERT ; R. SCHMIDT ; S. ZIEGLER ; F. GRIMMINGER ; M. YABUT ; B. TEMMESFELD ; D. WALMRATH ; H. MORR ; W. SEEGER.** Surfactant alterations in severe pneumonia, acute respiratory distress syndrome, and cardiogenic lung edema. *Am J Respir Crit Care Med,* 1996, vol. 153, 176-184
- **A. GÜNTHER ; R. SCHMIDT ; F. NIX ; M. YABUT-PEREZ ; C. GUTH ; S. ROSSEAU ; C. SIEBERT ; F. GRIMMINGER ; H. MORR ; H.G. VELCOVSKY.** Surfactant abnormalities in idiopathic pulmonary fibrosis, hypersensitivity pneumonitis and sarcoidosis. *Eur Respir J,* 1999, vol. 14, 565-573
- **HYUN-JEONG JEON ; YOUNG-II JEONG ; MI-KYEONG JANG ; YOUNG-HOON PARK ; JAE-WOON NAH.** Effect of solvent on the preparation of surfactant-free poly(DL-lactide-co-glycolide) nanoparticles and norfloxacin release characteristics. *Int J Pharm,* 2000, vol. 207, 99-108